(19) Europäisches Patentamt
European Patent Office
Office européen des brevets

(11) Veröffentlichungsnummer: **0 215 738**
**A1**

(12) **EUROPÄISCHE PATENTANMELDUNG**

(21) Anmeldenummer: **86810400.1**

(22) Anmeldetag: **03.09.86**

(51) Int. Cl.[4]: **C 07 D 401/14**, C 07 D 403/14, C 07 D 405/14, C 07 D 409/14, C 07 D 413/14, C 07 D 417/14, A 01 N 43/50

(30) Priorität: **09.09.85 CH 3881/85**
**27.02.86 CH 780/86**

(43) Veröffentlichungstag der Anmeldung: **25.03.87**
**Patentblatt 87/13**

(84) Benannte Vertragsstaaten: **BE CH DE FR GB IT LI NL**

(71) Anmelder: **CIBA-GEIGY AG, Klybeckstrasse 141, CH-4002 Basel (CH)**

(72) Erfinder: **Dürr, Dieter, Dr., Brändelistalweg 16, CH-4103 Bottmingen (CH)**
Erfinder: **Szczepanski, Henry, Dr., Bodenmatt, CH-4323 Wallbach (CH)**

(54) **Heterocyclylalkylester von 2-Imidazolinon-nicotinsäuren.**

(57) Neue Heterocyclylalkylester von 2-Imidazolinon-nicotinsäuren der untenstehenden Formel I haben gute pre- und postemergente selektivherbizide Eigenschaften, ferner beeinflussen, respektive hemmen sie das Pflanzenwachstum.

Die neuen Ester entsprechen der Formel I

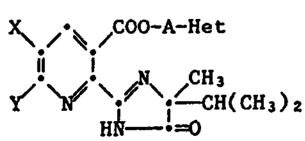

I

worin

A eine geradkettige oder verzweigte $C_1$-$C_4$-Alkylenbrükke X und Y unabhängig voneinander je Wasserstoff $C_1$-$C_3$-Alkyl, $C_1$-$C_3$-Haloalkyl, $C_1$-$C_3$-Alkoxy, $C_1$-$C_3$-Haloalkoxy, Halogen, oder X und Y bilden zusammen die Butadienbrücke, die durch Halogen, Cyan oder $C_1$-$C_4$-Alkyl substituiert sein kann,

Het einen 5-6 gliedrigen, ein bis 3 Stickstoffatome und/oder ein Sauerstoff- oder Schwefelatom und gegebenenfalls eine oder 2 Carbonylgruppen enthaltenden Heterocyclus, der benzanneliert und/oder durch Halogen, $C_1$-$C_3$-Alkyl, $C_1$-$C_3$-Halogenalkyl, $C_1$-$C_3$-Alkoxy, $C_1$-$C_3$-Halogenalkoxy, Nitro, Amino, $C_1$-$C_3$-Alkylamino oder $C_1$-$C_3$-Dialkylamino kann, bedeuten.

EP 0 215 738 A1

0215738

CIBA-GEIGY AG
Basel (Schweiz)

5-15500/1+2/=

# Heterocyclylalkylester von 2-Imidazolinon-nicotinsäuren

Die vorliegende Erfindung betrifft neue Heterocyclylalkylester von 2-Imidazolinon-nicotinsäuren mit herbizider und den Pflanzenwuchs regulierender Wirkung, sowie die Herstellung dieser neuen Verbindungen. Ferner betrifft sie Mittel, welche die neuen 2-Imidazolinon-nicotinsäureester enthalten sowie deren Verwendung zur selektiven Bekämpfung von Unkräutern oder zur Regulierung des Pflanzenwuchses.

Die neuen Heterocyclylalkylester von 2-Imidazolinon-nicotinsäuren entsprechen der Formel I

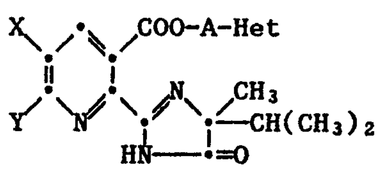

I

worin

A eine geradkettige oder verzweigte $C_1$-$C_4$-Alkylenbrücke

X und Y unabhängig voneinander je Wasserstoff $C_1$-$C_3$-Alkyl, $C_1$-$C_3$-Haloalkyl, $C_1$-$C_3$-Alkoxy , $C_1$-$C_3$-Haloalkoxy, Halogen, oder

X und Y bilden zusammen die Butadienbrücke CH=CH-CH=CH, die durch Halogen, Cyan oder $C_1$-$C_4$-Alkyl substituiert sein kann,

Het einen 5-6 gliedrigen, ein bis 3 Stickstoffatome und/oder ein Sauerstoff- oder Schwefelatom und gegebenenfalls eine oder 2 Carbonylgruppen enthaltenden Heterocyclus, der benzanneliert und/oder durch Halogen, $C_1$-$C_3$-Alkyl, $C_1$-$C_3$-Halogenalkyl, $C_1$-$C_3$-Alkoxy, $C_1$-$C_3$-Halogenalkoxy, Nitro, Amino, $C_1$-$C_3$-Alkylamino oder $C_1$-$C_3$-Dialkyamino substituiert sein kann, oder den

2-(4,5-Dihydro-3-methylthio-6-tert.-butyl-1,3,4-triazin-5-on-2-yl-hydrazinoxim)-furfur-5-yl-Rest bedeuten, wobei die Bestimmung gilt, dass Het nicht der unsubstituierte Furylrest sein darf.

In diesen Definitionen können die Alkylreste, wie auch die Alkylenreste, geradkettig oder verzweigt sein und die Bedeutung von Methyl, Methylen, Aethyl, Aethylen, Propyl, Propylen, Isopropyl, 1- oder 2-Methyläthylen, Butyl, Butylen, sec. Butyl, 1-Methylpropylen, Isobutyl, 2-Methylpropylen, tert.-Butyl, 2,2-Dimethyläthylen, 1,2-Dimethyläthylen, bedeuten.

Halogen bedeutet Fluor, Chlor, Brom oder Iod.

Die unter Het definierten Heterocyclen umfassen z.B. Pyrrol, Pyrrolidin, Pyrrolidinon, Pyrrolinon, Pyrrolindion, Pyridin, Pyridinon, Pyridindion, Piperidin, Piperidinon, Imidazol, Imidazolinon, Pyrazol, Pyrazolon, Piperazin, Diazin, Diazinon, Piperazinon, Triazol, Triazolon, Triazin, Triazinon, Benzazol, Indol, Chinolin, Chinolinon, Benzdiazol, Benzdiazolon, Furfuryl, Tetrahydrofuran, Pyran, Oxazol, Oxadiazol, Morpholin, Thiophen, Thiazol, Thiadiazol sowie Thiomorpholin.

Dabei gilt die Bestimmung, dass Het nicht Furan ist.

Diese Ringe können unsubstituiert oder durch Halogen, $C_1$-$C_3$-Alkyl, $C_1$-$C_3$-Halogenalkyl, $C_1$-$C_3$-Alkoxy, $C_1$-$C_3$-Halogenalkoxy, Nitro, Amino oder $C_1$-$C_3$-Alkylamino resp. $C_1$-$C_3$-Dialkyamino substituiert sein.

Die Herstellung solcher Verbindungen lässt sich durch folgendes Schema darstellen:

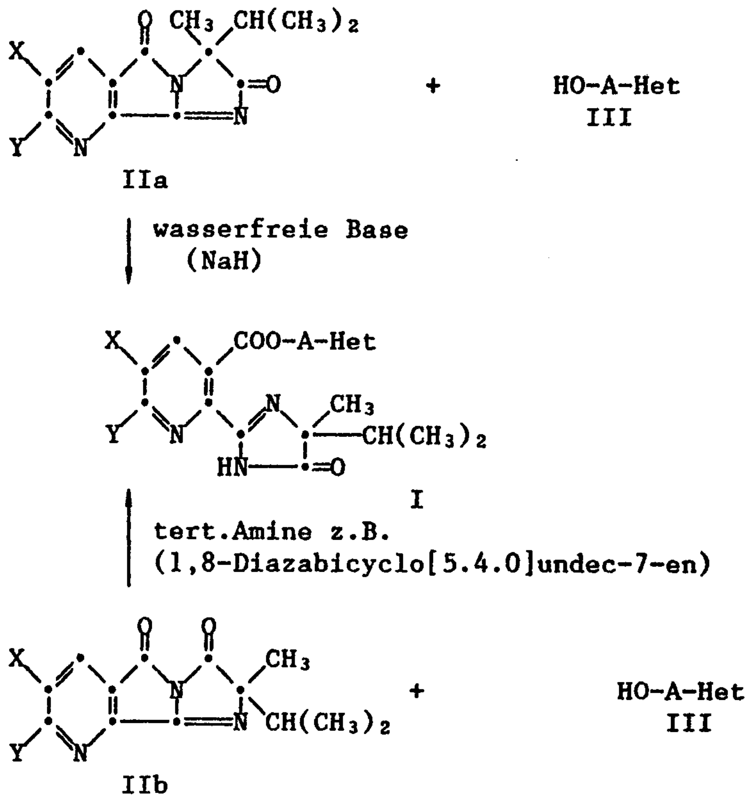


Das erfindungsgemässe Verfahren zur Herstellung der Imidazolinon-Verbindungen der Formel I besteht darin, dass man eine Verbindung der Formel IIa resp. IIb

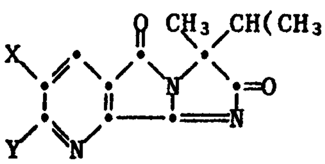
(IIa)

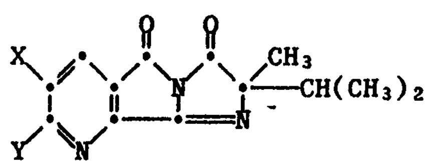
(IIb)

worin X und Y die unter der Formel I gegebene Bedeutung haben, in einem inerten organischen Lösungs- oder Verdünnungsmittel, in Gegenwart eines basischen Mittels mit einem Alkanol der Formel III umsetzt,

HO-A-Het (III)

worin A und Het die unter der Formel I angegebene Bedeutung haben.

Als Lösungsmittel für diese Umsetzungen eignen sich möglichst wasserfreie Kohlenwasserstoffe, Aether oder Ketone, wie z.B. Benzol, Toluol, Xylol, Hexan, Cyclohexan, Diisopropyläther, Tetrahydrofuran, Dioxan.

Als basische Mittel kommen z.B. Natriumhydrid, 1,8-Diaza-bicyclo-[5.4.0]undec-7-en, und Triäthylamin in Frage.

Diese Reaktionen werden bei Temperaturen zwischen 0 und 200° durchgeführt, im allgemeinen beim Siedepunkt des Reaktionsgemisches.

Ausgangsprodukte der Formel IIa sind bekannt und oder nach bekannten Methoden herstellbar, z.B. gemäss der Europäischen Offenlegungsschrift EP-A 41 623 indem man unter basischen Bedingungen ein N-(α-Isopropyl-α-methylacetamido)-2,3-pyridincarbonsäureimid kondensiert gemäss dem Schema

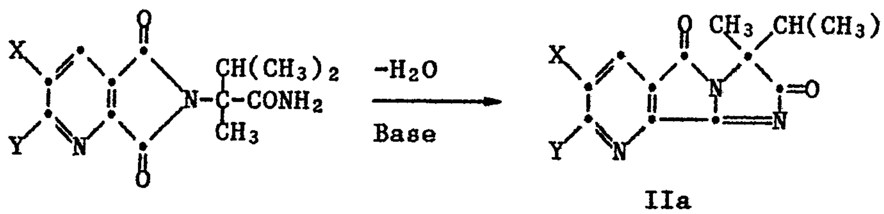


IIa

Ein Pyridin-2,3-dicarbonsäure-α-isopropyl-α-methyl-acetonitril der Formel IV kann gemäss einem einfachen Verfahren hergestellt werden, indem man ein ungesättigtes Hydrazon mit einem 2-Chlor- oder 2-Brom-N(α-isopropyl-α-methyl-acetonitril)maleinimid kondensiert gemäss dem Schema

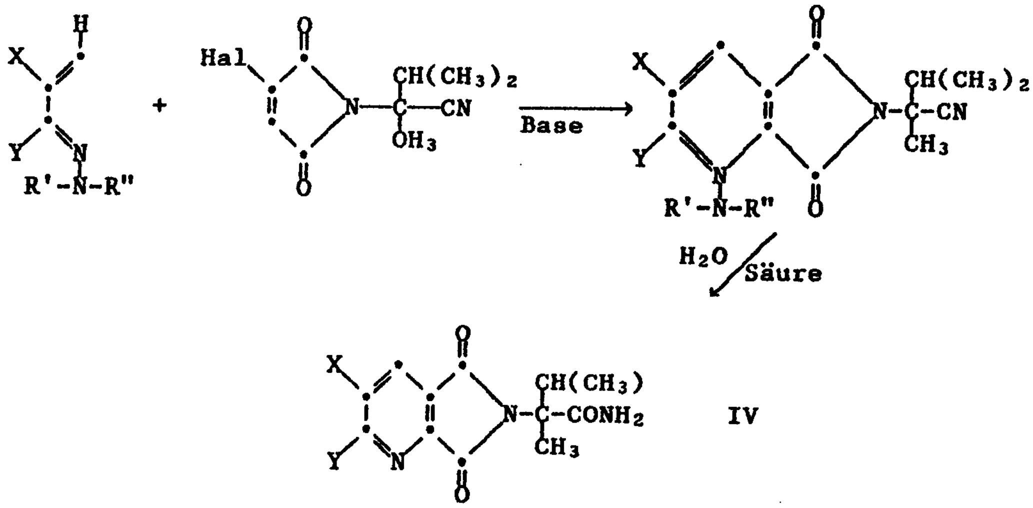


IV

In diesen Formeln bedeuten R' und R" je Wasserstoff oder $C_1$-$C_4$-Alkyl, Hal Chlor oder Brom während X und Y die unter der Formel I gegebene Bedeutung haben.

Die Ausgangsstoffe der Formel IIb werden erhalten, indem man das obige N-(α-Isopropyl-α-methylacetamido)-2,3-pyridincarbonsäureimid in Gegenwart einer Base wie z.B. Natronlauge zum 2-(4-Isopropyl-4-methyl-5-oxo-imidazolidin)-nicotinsäurederivat umwandelt,

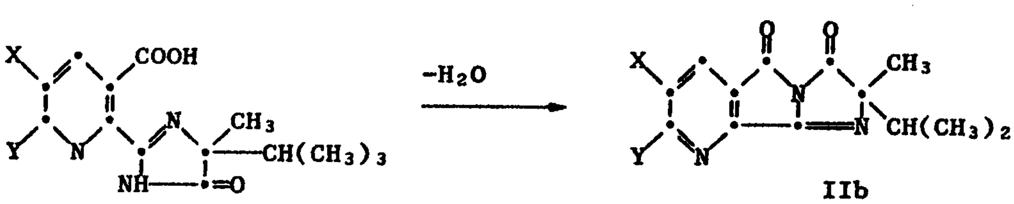


IIb

welches sich beim Behandeln mit einem wasserentziehenden Mittel in einem inerten organischen Lösungsmittel unter Verlust eines Wassermoleküls zum Ausgangsprodukt der Formel IIb (2-Isopropyl-2-methyl-3H-imidazo [1',2':1,2]pyrrolo[3,4-b]pyridin 3,5-dion) umwandelt. Solche Nicotinsäureester und ihre Herstellung sind auch in der Europäischen Offenlegungsschrift EP-A 41 623 beschrieben.

Als wasserentziehende Mittel für diese Cyclisation kommen z.B. Kochen am Wasserabschneider, eine molare Menge einer starken Säure z.B. konzentrierter Schwefelsäure oder eines Anhydrides oder ein

wasserabsorbierende Reagentien wie Cyclohexancarbodiimid, Thionylchlorid oder Phosgen in Gegenwart von etwas Dimethylformamid in Frage.

Die Reaktionen werden soweit sie sich nicht schon bei Raumtemperatur durchführen lassen bei Temperaturen zwischen 0°C und 200°C durchgeführt, d.h. man erhitzt - falls nötig - bis zum Siedepunkt des Reaktionsgemisches und kühlt wenn nötig mit Eis/Wasser oder Eis/Solebad ab.

Als Basen kommen für diese Reaktionen vor allem anorganische Basen wie Natriumhydroxid, Natriumcarbonat, Natriumhydrid, Calciumhydroxid, Calciumcarbonat, Kaliumhydroxid, Kaliumcarbonat, Ammoniak sowie tertiäre inorganische Basen wie Triäthylamin in Frage.

Geeignete Lösungsmittel sind z.B. polare, aprotische Lösungsmittel, die alleine oder in Mischungen aus mindestens zwei Lösungsmitteln verwendet werden können.

Unter den neuen Estern der Formel I haben sich die Nicotinsäureester als sehr aktiv erwiesen, in denen
A eine Methylen oder Aethylenbrücke, die durch Methyl substituiert sein kann,
X Wasserstoff oder $C_1$-$C_3$-Alkyl,
Y Wasserstoff und
Het die oben gegebenen Bedeutung haben,
bedeuten, ferner solche, in denen
A die Methylen-, Aethyl-1-en oder Aethyl-2-en-brücke,
X Wasserstoff oder $C_1$-$C_3$-Alkyl
Y Wasserstoff und
Het einen ungesättigten 5-6-gliedrigen, ein bis drei Stickstoffatome und gegebenenfalls eine oder zwei Carbonylgruppen enthaltenden Heterocyclus, der benzoannelliert und durch Methyl substituiert sein kann, bedeuten.

Insbesondere waren folgende Verbindungen gut wirksam:

2-(5-Isopropyl-5-methyl-4-oxo-4,5-dihydroimidazolin-2-yl)-5-methyl-nicotinsäure-(4-pyridylmethyl)ester;

5-Aethyl-2-(5-isopropyl-5-methyl-4-oxo-4,5-dihydro-imidazolin-2-yl)-nicotinsäureindolyläthyl-ester;

2-(5-Isopropyl-5-methyl-4-oxo-4,5-dihydroimidazolin-2-yl)-5-methyl-nicotinsäure-indolyläthyl-ester;

5-Aethyl-2-(5-isopropyl-5-methyl-4-oxo-4,5-dihydro-imidazolin-2-yl)-nicotinsäure-pyridin-2-yläthylester;

5-Aethyl-2-(5-isopropyl-5-methyl-4-oxo-4,5-dihydro-imidazolin-2-yl)-nicotinsäure-isoindol-2-yläthyl-ester;

5-Aethyl-2-(5-isopropyl-5-methyl-4-oxo-4,5-dihydro-imidazolin-2-yl)-nicotinsäure-pyridin-2-ylmethyl-ester;

5-Aethyl-2-(5-isopropyl-5-methyl-4-oxo-4,5-dihydro-imidazolin-2-yl)-nicotinsäure-pyridin-3-ylmethyl-ester;

5-Aethyl-2-(5-isopropyl-5-methyl-4-oxo-4,5-dihydro-imidazolin-2-yl)-nicotinsäure-pyridin-4-ylmethyl-ester;

2-(5-Isopropyl-5-methyl-4-oxo-4,5-dihydro-imidazolin-2-yl)-5-methyl-nicotinsäure-pyridin-2-ylmethyl-ester;

2-(5-Isopropyl-5-methyl-4-oxo-4,5-dihydro-imidazolin-2-yl)-5-methyl-nicotinsäure-pyridin-3-ylmethyl)-ester

2-(5-Isopropyl-5-methyl-4-oxo-4,5-dihydro-imidazolin-2-yl)-nicotinsäure-pyridin-2-yläthylester und

2-(5-Isopropyl-5-methyl-4-oxo-4,5-dihydro-imidazolin-2-yl)-5-methyl-nicotinsäure-pyridin-2-yläthylester.

Die Erfindung betrifft alle diastereomeren und enantiomeren Isomeren der Verbindungen der Formel I.

Die Wirkstoffe der Formel I werden in der Regel mit Aufwandmengen von 0,05 bis 4 kg/ha insbesondere 0,1 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von

natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlensotffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykol-äthergruppen enthaltenden Polyäthylenoxidaddukte an Polypropylen-

glykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
Dr. Helmut Stache "Tensid Taschenbuch"
Carl Hanser Verlag, München/Wien 1981.

Die gebrauchsfertigen Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | | |
|---|---|---|
| Wirkstoff der Formel I: | 1 bis 20 % bevorzugt | 5 bis 10 % |
| oberflächenaktives Mittel: | 5 bis 30 %,vorzugsweise | 10 bis 20 % |
| flüssiges Trägermittel: | 50 bis 94 %,vorzugsweise | 70 bis 85 %. |

Stäube:

| | | |
|---|---|---|
| Wirkstoff der Formel I: | 0,1 bis 10 %, | |
| vorzugsweise | 0,1 bis 1 % | |
| festes Trägermittel: | 99,9 bis 90 %, vorzugsweise | 99,9 bis 99 %. |

Suspensions-Konzentrate:

| | | |
|---|---|---|
| Wirkstoff der Formel I: | 5 bis 75 %, vorzugsweise | 10 bis 50 % |
| Wasser: | 94 bis 25 %, vorzugsweise | 90 bis 30 % |
| oberflächenaktives Mittel: | 1 bis 40 %, vorzugsweise | 2 bis 30 %. |

Benetzbare Pulver:

| | | |
|---|---|---|
| Wirkstoff der Formel I: | 0,5 bis 90 %, vorzugsweise | 1 bis 80 % |
| oberflächenaktives Mittel: | 0,5 bis 20 %, vorzugsweise | 1 bis 15 % |
| festes Trägermittel: | 5 bis 95 %, vorzugsweise | 15 bis 90 %. |

Granulate:

| | | |
|---|---|---|
| Wirkstoff der Formel I: | 0,5 bis 30 %, vorzugsweise | 3 bis 15 % |
| festes Trägermittel: | 99,5 bis 70 %, vorzugsweise | 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Das nachfolgende Beispiel veranschaulicht die Herstellung einer Verbindung der Formel I. Weitere in entsprechender Weise hergestellte Wirkstoffe sind in den anschliessenden Tabellen aufgeführt. Temperaturangaben beziehen sich auf Celsius-Grade, Drucke sind in Millibar angegeben.

<u>Beispiel 1:</u> Herstellung von 2-(5-Isopropyl-5-methyl-4-oxo-4,5-dihydro-imidazolin-2-yl)-5-methyl-nicotinsäure-(4-pyridylmethyl)-ester

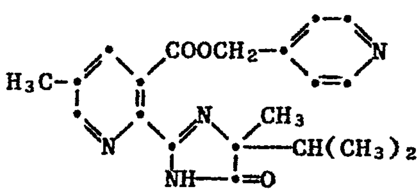

7,71 g (=0,03 M) 3,5-Dioxo-2-isopropyl-2,8-dimethyl-2,3-dihydro-imidazo[1',2':1,2]-5H-pyrrolo [3,4-b]pyridin, 3,27 g 4-Hydroxymethyl-pyridin und 4 Tropfen 1,8-Diazabicyclo[5.4.0]undec-7-en (DBU) werden in 150 ml Toluol über Nacht auf Rückfluss erhitzt. Man dampft dann das Lösungsmittel ab und kristallisiert den Rückstand aus Methylenchlorid/Hexan. Ausbeute 9,5 g (=87 % der Theorie). Schmelzpunkt 182-184°.

Das als Ausgangsmaterial benötigte 3,5-Dioxo-2-isopropyl-2,8-dimethyl-2,3-dihydro-imidazo[1',2':1,2]5-H-pyrrolo[3,4-b]pyridin wird wie folgt hergestellt:

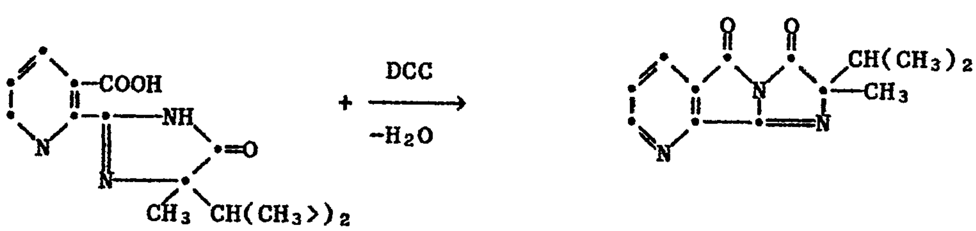

Zu einer Lösung von 11,1 g Dicyclohexycarbodiimid (DCC) in 100 ml Methylenchlorid gibt man unter Rühren 13,1 g (0.05 Mol) 2-(5-Isopropyl-5-methyl-4-oxo-4,5-dihydro-imidazolin-2-yl)-nicotinsäure und rührt zwei Stunden bei Raumtemperatur weiter. Dann nutscht man den entstandenen Dicyclohexylharnstoff ab und wäscht den Rückstand mit wenig Methylenchlorid nach. Das Filtrat wird eingedampft und der Rückstand aus Essigsäureäthylester/Hexan umkristallisiert. Man erhält so das Titelprodukt in 86%iger Ausbeute (10.4 g) als Kristalle, die bei 132-133° schmelzen.

In analoger Weise dazu werden die folgenden Ausgangsprodukte erhalten.

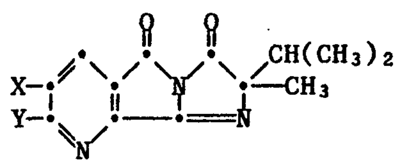

| X | X | phys. Daten |
|---|---|---|
| $nC_3H_7$ | H | Smp. 132-133° |
| $CH(CH_3)_2$ | H | Smp. 145-146° |
| $C_2H_5$ | H | Smp. 136-138° |
| $n-C_4H_9$ | H | Smp. 116-119° |
| $CH_3$ | H | Smp. 129-131° |

0215738

Auf dieselbe Weise werden bei Einsatz der entsprechenden Ausgangsprodukte die folgenden Verbindungen erhalten:

Tabelle 1:

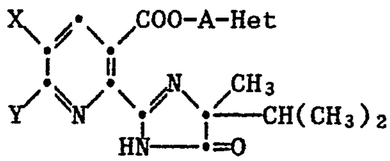

| No. | X | Y | A | Het | Smp. |
|---|---|---|---|---|---|
| 1.001 | $CH_3$ | H | $CH_2$ | Pyridin-4-yl | 182-184° Beisp.1 |
| 1.002 | $CH_3$ | H | $CH_2CH_2$ | Indol-2-yl | 146-149° |
| 1.003 | $C_2H_5$ | H | $CH_2CH_2$ | Indol-2-yl | 166-168° |
| 1.004 | $C_2H_5$ | H | $CH_2CH_2$ | Pyridin-2-yl | Harz |
| 1.005 | $C_2H_5$ | H | $CH_2CH_2$ | Isoindol-1,3-dion-2-yl | 130-131° |
| 1.006 | $C_2H_5$ | H | $CH_2$ | Pyridin-2-yl | 80-82° |
| 1.007 | $C_2H_5$ | H | $CH_2$ | Pyridin-3-yl | 123-124° |
| 1.008 | $C_2H_5$ | H | $CH_2$ | Pyridin-4-yl | 135-137° |
| 1.009 | $CH_3$ | H | $CH_2$ | Pyridin-3-yl | 133-134° |
| 1.010 | $CH_3$ | H | $CH_2$ | Pyridin-2-yl | 172-173° |
| 1.011 | H | H | $CH_2CH_2$ | Pyridin-2-yl | 105-106° |
| 1.012 | $CH_3$ | H | $CH_2CH_2$ | Pyridin-2-yl | 119-122° |
| 1.013 | H | H | $CH(CH_3)$ | Pyridin-4-yl | |
| 1.014 | $(CH=CH)_2$ | | $CH_2CH_2$ | Pyridin-4-yl | |
| 1.015 | $(CH=CH)_2$ | | $CH_2$ | Pyridin-4-yl | |
| 1.016 | $(CH=CH)_2$ | | $CH_2$ | Pyridazin-2-yl | |
| 1.017 | $(CH=CH)_2$ | | $CH_2$ | Pyrrol-1-yl | |
| 1.018 | H | H | $CH_2CH_2$ | Pyrrol-1-yl | |
| 1.019 | H | H | $CH_2$ | Pyrrol-1-yl | |
| 1.020 | H | H | $CH_2CH_2$ | Imidazol-1-yl | |
| 1.021 | $CH_3$ | H | $CH_2$ | Imidazol-1-yl | |
| 1.022 | H | H | $CH_2$ | 1H-1,2,4-Triazol-1-yl | |
| 1.023 | $CH_3$ | H | $CH_2CH_2$ | 1H-1,2,4-Triazol-1-yl | |
| 1.024 | $CH_3$ | H | $CH_2$ | 1H-1,3,4-Triazol-1-yl | |
| 1.025 | H | H | $CH_2CH_2$ | 1H-1,3,4-Triazol-1-yl | |
| 1.026 | H | H | $CH_2$ | Pyrimidine-4-yl | |

| No. | X | Y | A | Het | Smp. |
|---|---|---|---|---|---|
| 1.027 | $CH_3$ | H | $CH(CH_3)CH_2$ | Pyrimidine-4-yl | |
| 1.028 | $CH_3$ | H | $CH_2$ | Pyrimidine-2-yl | |
| 1.029 | $C_2H_5$ | H | $CH_2$ | Pyrimidine-2-yl | |
| 1.030 | H | H | $CH_2CH_2$ | Indol-3-yl | |
| 1.031 | $CH_3$ | H | $CH_2$ | Indol-3-yl | |
| 1.032 | $(CH=CH)_2$ | | $CH_2$ | 4-Methylpyridin-2-yl | |
| 1.033 | $(CH=CH)_2$ | | $CH_2$ | Pyrimidine-2-yl | |
| 1.034 | $(CH=CH)_2$ | | $CH_2$ | Indol-2-yl | |
| 1.035 | $CH_3$ | H | $CH_2$ | Tetrahydrofur-2-yl | 113-115° |
| 1.036 | $CH_3$ | H | $CH_2CH_2$ | Indol-2-yl | 146-149° |
| 1.037 | H | H | $-C_3H_6-$ | Pyrazol-4-yl | 168-172° |
| 1.038 | $CH_3$ | H | $CH_2CH_2$ | Phthalimido | 151-153° |
| 1.039 | $C_2H_5$ | H | $CH_2$ | Furfurol-5-yl | Harz |
| 1.040 | H | H | $C_3H_6$ | 1,2-Oxazol-4-yl | Oel |
| 1.041 | H | H | $CH_2$ | Pyridin-2-yl | 111-112° |
| 1.042 | H | H | $CH_2$ | Pyridin-3-yl | 137-138° |
| 1.043 | H | H | $CH_2$ | Pyridin-4-yl | 155-157° |
| 1.044 | H | H | $CH_2CH_2$ | Indol-2-yl | Harz |
| 1.045 | H | H | $CH_2CH_2$ | Phthalimido | Harz |
| 1.046 | H | H | $CH_2$ | Tetrahydrofur-2-yl | |
| 1.047 | H | H | $C_3H_6$ | 1-Phenylpyrazol-4-yl | 118-119° |
| 1.048 | H | H | $C_3H_6$ | 1-Methylpyrazol-4-yl | 134-135° |
| 1.049 | H | H | $C_3H_6$ | 1-Aethylpyrazol-4-yl | 87-88° |
| 1.050 | $C_2H_5$ | H | $C_3H_6$ | 1-Methylpyrazol-4-yl | 90-92° |
| 1.051 | $C_2H_5$ | H | $C_3H_6$ | 1,2-Oxazol-4-yl | 90-92° |
| 1.052 | $C_2H_5$ | H | $C_3H_6$ | 1-Aethylpyrazol-4-yl | Harz |
| 1.053 | $(CH=CH)_2$ | | $CH_2$ | 1-Aethylpyrazol-4-yl | Harz |
| 1.054 | $C_3H_7n$ | H | $CH_2$ | Pyridin-4-yl | 110° |
| 1.055 | $C_3H_7n$ | H | $C_2H_4$ | Pyridin-2-yl | 108° |
| 1.056 | $C_3H_7n$ | H | $CH_2$ | Tetrahydrofur-2-yl | 87-90° |
| 1.057 | H | H | $CH_2CH_2$ | 1,2-Oxazol-4-yl | Harz |
| 1.058 | $CH_3$ | H | $CH_2CH_2$ | 1,2-Oxazol-4-yl | Oel |
| 1.059 | $C_2H_5$ | H | $CH_2CH_2$ | 1,2-Oxazol-4-yl | Oel |
| 1.060 | $(CH=CH)_2$ | | $CH_2CH_2$ | 1,2-Oxazol-4-yl | |

| No. | X | Y | A | Het | Smp. |
|---|---|---|---|---|---|
| 1.061 | Cl | H | $CH_2CH_2$ | 1,2-Oxazol-4-yl | |
| 1.062 | CH=CCl-CH=CH | | $CH_2CH_2$ | 1,2-Oxazol-4-yl | |
| 1.063 | Br | H | $CH_2CH_2$ | 1,2-Oxazol-4-yl | |
| 1.064 | | $CH(CH_3)_2$ | $CH_2CH_2$ | 1,2-Oxazol-4-yl | |
| 1.065 | H | H | $CH_2CH_2$ | Pyrazol-4-yl | |
| 1.066 | H | H | $CH_2CH_2$ | 1-Methyl-pyrazol-4-yl | |
| 1.067 | H | H | $CH_2CH_2$ | 1-Aethyl-pyrazol-4-yl | |
| 1.068 | H | H | $CH_2CH_2$ | 1-Phenyl-pyrazol-4-yl | 131-132° |
| 1.069 | $CH_3$ | H | $CH_2CH_2$ | Pyrazol-4-yl | |
| 1.070 | $CH_3$ | H | $CH_2CH_2$ | 1-Methyl-pyrazol-4-yl | |
| 1.071 | $CH_3$ | H | $CH_2CH_2$ | 1-Aethyl-pyrazol-4-yl | |
| 1.072 | $CH_3$ | H | $CH_2CH_2$ | 1-Phenyl-pyrazol-4-yl | |
| 1.073 | $C_2H_5$ | H | $CH_2CH_2$ | Pyrazol-1-yl | 148-151° |
| 1.074 | $C_2H_5$ | H | $CH_2CH_2$ | 1-Methyl-pyrazol-4-yl | |
| 1.075 | $C_2H_5$ | H | $CH_2CH_2$ | 1-Aethyl-pyrazol-4-yl | |
| 1.076 | $C_2H_5$ | H | $CH_2CH_2$ | 1-Phenyl-pyrazol-4-yl | glasartig |
| 1.077 | $(CH=CH)_2$ | | $CH_2CH_2$ | Pyrazol-4-yl | |
| 1.078 | $(CH=CH)_2$ | | $CH_2CH_2$ | 1-Methyl-pyrazol-4-yl | |
| 1.079 | $(CH=CH)_2$ | | $CH_2CH_2$ | 1-Aethyl-pyrazol-4-yl | |
| 1.080 | $(CH=CH)_2$ | | $CH_2CH_2$ | 1-Phenyl-pyrazol-4-yl | fest |
| 1.081 | Cl | H | $CH_2CH_2$ | Pyrazol-4-yl | |
| 1.082 | Cl | H | $CH_2CH_2$ | 1-Methyl-pyrazol-4-yl | |
| 1.083 | Cl | H | $CH_2CH_2$ | 1-Aethyl-pyrazol-4-yl | |
| 1.084 | Cl | H | $CH_2CH_2$ | 1-Phenyl-pyrazol-4-yl | |
| 1.085 | CH=CCl-CH=CH | | $CH_2CH_2$ | Pyrazol-4-yl | |
| 1.086 | CH=CCl-CH=CH | | $CH_2CH_2$ | 1-Methylpyrazol-4-yl | |
| 1.087 | CH=CCl-CH=CH | | $CH_2CH_2$ | 1-Aethyl-pyrazol-4-yl | |
| 1.088 | CH=CCl-CH=CH | | $CH_2CH_2$ | 1-Phenyl-pyrazol-4-yl | |
| 1.089 | Br | H | $CH_2CH_2$ | Pyrazol-4-yl | |
| 1.090 | Br | H | $CH_2CH_2$ | 1-Methyl-pyrazol-4-yl | |
| 1.091 | Br | H | $CH_2CH_2$ | 1-Aethyl-pyrazol-4-yl | |
| 1.092 | Br | H | $CH_2CH_2$ | 1-Phenyl-pyrazol-4-yl | |
| 1.093 | H | $CH(CH_3)_2$ | $CH_2CH_2$ | Pyrazol-4-yl | |
| 1.094 | H | $CH(CH_3)_2$ | $CH_2CH_2$ | 1-Methylpyrazol-4-yl | |

| No. | X | Y | A | Het | Smp. |
|---|---|---|---|---|---|
| 1.095 | H | $CH(CH_3)_2$ | $CH_2CH_2$ | 1-Aethylpyrazol-4-yl | |
| 1.096 | H | $CH(CH_3)_2$ | $CH_2CH_2$ | 1-Phenyl-pyrazol-4-yl | |
| 1.097 | H | H | $CH_2CH_2$ | 3,5-Dibromoxazol-4-yl | |
| 1.098 | $CH_3$ | H | $CH_2CH_2$ | 3,5-Dibromoxazol-4-yl | |
| 1.099 | $C_2H_5$ | H | $CH_2CH_2$ | 3,5-Dibromoxazol-4-yl | |
| 1.100 | $(CH=CH)_2$ | | $CH_2CH_2$ | 3,5-Dibromoxazol-4-yl | |
| 1.101 | Cl | H | $CH_2CH_2$ | 3,5-Dibromoxazol-4-yl | |
| 1.102 | CH=CCl-CH=CH | | $CH_2CH_2$ | 3,5-Dibromoxazol-4-yl | |
| 1.103 | Br | H | $CH_2CH_2$ | 3,5-Dibromoxazol-4-yl | |
| 1.104 | H | $CH(CH_3)_2$ | $CH_2CH_2$ | 3,5-Dibromoxazol-4-yl | |
| 1.105 | H | H | $CH_2$ | Benzimidazol-2-yl | |
| 1.106 | $CH_3$ | H | $CH_2$ | Benzimidazol-2-yl | |
| 1.107 | $C_2H_5$ | H | $CH_2$ | Benzimidazol-2-yl | |
| 1.108 | $(CH=CH)_2$ | | $CH_2$ | Benzimidazol-2-yl | |
| 1.109 | Cl | H | $CH_2$ | Benzimidazol-2-yl | |
| 1.110 | CH=CCl-CH=CH | | $CH_2$ | Benzimidazol-2-yl | |
| 1.111 | Br | H | $CH_2$ | Benzimidazol-2-yl | |
| 1.112 | H | $CH(CH_3)_2$ | $CH_2$ | Benzimidazol-2-yl | |
| 1.113 | H | H | $CH_2$ | Imidazol-2-yl | |
| 1.114 | $CH_3$ | H | $CH_2$ | Imidazol-2-yl | |
| 1.115 | $C_2H_5$ | H | $CH_2$ | Imidazol-2-yl | |
| 1.116 | $(CH=CH)_2$ | | $CH_2$ | Imidazol-2-yl | |
| 1.117 | Cl | H | $CH_2$ | Imidazol-2-yl | |
| 1.118 | CH=CCl-CH=CH | | $CH_2$ | Imidazol-2-yl | |
| 1.119 | Br | H | $CH_2$ | Imidazol-2-yl | |
| 1.120 | H | $CH(CH_3)_2$ | $CH_2$ | Imidazol-2-yl | |
| 1.121 | H | H | $CH_2$ | Benzoxazol-2-yl | |
| 1.122 | $CH_3$ | H | $CH_2$ | Benzoxazol-2-yl | |
| 1.123 | $C_2H_5$ | H | $CH_2$ | Benzoxazol-2-yl | |
| 1.124 | $(CH=CH)_2$ | | $CH_2$ | Benzoxazol-2-yl | |
| 1.125 | Cl | H | $CH_2$ | Benzoxazol-2-yl | |
| 1.126 | CH=CCl-CH=CH | | $CH_2$ | Benzoxazol-2-yl | |
| 1.127 | Br | H | $CH_2$ | Benzoxazol-2-yl | |
| 1.128 | H | $CH(CH_3)_2$ | $CH_2$ | Benzoxazol-2-yl | |

| No. | X | Y | A | Het | Smp. |
|---|---|---|---|---|---|
| 1.129 | H | H | $CH(CH_3)CH_2$ | 3-Hydroxy-1,2-oxazol-5-yl | |
| 1.130 | $CH_3$ | H | $CH(CH_3)CH_2$ | 3-Hydroxy-1,2-oxazol-5-yl | |
| 1.131 | $C_2H_5$ | H | $CH(CH_3)CH_2$ | 3-Hydroxy-1,2-oxazol-5-yl | |
| 1.132 | $(CH{=}CH)_2$ | | $CH(CH_3)CH_2$ | 3-Hydroxy-1,2-oxazol-5-yl | |
| 1.133 | Cl | H | $CH(CH_3)CH_2$ | 3-Hydroxy-1,2-oxazol-5-yl | |
| 1.134 | CH=CCl-CH=CH | | $CH(CH_3)CH_2$ | 3-Hydroxy-1,2-oxazol-5-yl | |
| 1.135 | Br | H | $CH(CH_3)CH_2$ | 3-Hydroxy-1,2-oxazol-5-yl | |
| 1.136 | H | $CH(CH_3)_2$ | $CH(CH_3)CH_2$ | 3-Hydroxy-1,2-oxazol-5-yl | |
| 1.137 | H | H | $(CH_2)_4$ | 2-Methyl-pyridin-4-yl | |
| 1.138 | $CH_3$ | H | $(CH_2)_4$ | 2-Methyl-pyridin-4-yl | |
| 1.139 | $C_2H_5$ | H | $(CH_2)_4$ | 2-Methyl-pyridin-4-yl | |
| 1.140 | $(CH{=}CH)_2$ | | $(CH_2)_4$ | 2-Methyl-pyridin-4-yl | |
| 1.141 | Cl | H | $(CH_2)_4$ | 2-Methyl-pyridin-4-yl | |
| 1.142 | CH=CCl-CH=CH | | $(CH_2)_4$ | 2-Methyl-pyridin-4-yl | |
| 1.143 | Br | H | $(CH_2)_4$ | 2-Methyl-pyridin-4-yl | |
| 1.144 | H | $CH(CH_3)_2$ | $(CH_2)_4$ | 2-Methyl-pyridin-4-yl | |
| 1.145 | H | H | $CH_2$ | 3-Methyl-1,2,4-triazol-5-yl | |
| 1.146 | $CH_3$ | H | $CH_2$ | 3-Methyl-1,2,4-triazol-5-yl | |
| 1.147 | $C_2H_5$ | H | $CH_2$ | 3-Methyl-1,2,4-triazol-5-yl | |
| 1.148 | $(CH{=}CH)_2$ | | $CH_3$ | 3-Methyl-1,2,4-triazol-5-yl | |
| 1.149 | Cl | H | $CH_2$ | 3-Methyl-1,2,4-triazol-5-yl | |
| 1.150 | CH=CCl-CH=CH | | $CH_2$ | 3-Methyl-1,2,4-triazol-5-yl | |
| 1.151 | Br | H | $CH_2$ | 3-Methyl-1,2,4-triazol-5-yl | |
| 1.152 | H | $CH(CH_3)_2$ | $CH_2$ | 3-Methyl-1,2,4-triazol-5-yl | |
| 1.153 | H | H | $CH_2$ | 1,3,4-Oxadiazol-2-yl | |
| 1.154 | $CH_3$ | H | $CH_2$ | 1,3,4-Oxadiazol-2-yl | |
| 1.155 | $C_2H_5$ | H | $CH_2$ | 1,3,4-Oxadiazol-2-yl | |
| 1.156 | $(CH{=}CH)_2$ | | $CH_2$ | 1,3,4-Oxadiazol-2-yl | |
| 1.157 | Cl | H | $CH_2$ | 1,3,4-Oxadiazol-2-yl | |
| 1.158 | CH=CCl-CH=CH | | $CH_2$ | 1,3,4-Oxadiazol-2-yl | |
| 1.159 | Br | H | $CH_2$ | 1,3,4-Oxadiazol-2-yl | |
| 1.160 | H | $CH(CH_3)_2$ | $CH_2$ | 1,3,4-Oxadiazol-2-yl | |
| 1.161 | H | H | $CH_2$ | 4-Nitro-pyridin-2-yl | |
| 1.162 | $CH_3$ | H | $CH_2$ | 4-Nitro-pyridin-2-yl | |

| No. | X | Y | A | Het | Smp. |
|---|---|---|---|---|---|
| 1.163 | $C_2H_5$ | H | $CH_2$ | 4-Nitro-pyridin-2-yl | |
| 1.164 | $(CH=CH)_2$ | H | $CH_2$ | 4-Nitro-pyridin-2-yl | |
| 1.165 | Cl | H | $CH_2$ | 4-Nitro-pyridin-2-yl | |
| 1.166 | CH=CCl-CH=CH | | $CH_2$ | 4-Nitro-pyridin-2-yl | |
| 1.167 | Br | H | $CH_2$ | 4-Nitro-pyridin-2-yl | |
| 1.168 | H | $CH(CH_3)_2$ | $CH_2$ | 4-Nitro-pyridin-2-yl | |
| 1.169 | H | H | $CH_2$ | 4-Chlor-pyridin-2-yl | |
| 1.170 | $CH_3$ | H | $CH_2$ | 4-Chlor-pyridin-2-yl | |
| 1.171 | $C_2H_5$ | H | $CH_2$ | 4-Chlor-pyridin-2-yl | |
| 1.172 | $(CH=CH)_2$ | H | $CH_2$ | 4-Chlor-pyridin-2-yl | |
| 1.173 | Cl | H | $CH_2$ | 4-Chlor-pyridin-2-yl | |
| 1.174 | CH=CCl-CH=CH | | $CH_2$ | 4-Chlor-pyridin-2-yl | |
| 1.175 | Br | H | $CH_2$ | 4-Chlor-pyridin-2-yl | |
| 1.176 | H | $CH(CH_3)_2$ | $CH_2$ | 4-Chlor-pyridin-2-yl | |
| 1.177 | H | H | $CH_2$ | 2-Chlor-pyridin-6-yl | |
| 1.178 | $CH_3$ | H | $CH_2$ | 2-Chlor-pyridin-6-yl | |
| 1.179 | $C_2H_5$ | H | $CH_2$ | 2-Chlor-pyridin-6-yl | |
| 1.180 | $(CH=CH)_2$ | | $CH_2$ | 2-Chlor-pyridin-6-yl | |
| 1.181 | Cl | H | $CH_2$ | 2-Chlor-pyridin-6-yl | |
| 1.182 | CH=CCl-CH=CH | | $CH_2$ | 2-Chlor-pyridin-6-yl | |
| 1.183 | Br | H | $CH_2$ | 2-Chlor-pyridin-6-yl | |
| 1.184 | H | $CH(CH_3)_2$ | $CH_2$ | 2-Chlor-pyridin-6-yl | |
| 1.185 | H | H | $CH_2$ | Chinolin-2-yl | |
| 1.186 | $CH_3$ | H | $CH_2$ | Chinolin-2-yl | |
| 1.187 | $C_2H_5$ | H | $CH_2$ | Chinolin-2-yl | |
| 1.188 | $(CH=CH)_2$ | | $CH_2$ | Chinolin-2-yl | |
| 1.189 | Cl | H | $CH_2$ | Chinolin-2-yl | |
| 1.190 | CH=CCl-CH=CH | | $CH_2$ | Chinolin-2-yl | |
| 1.191 | Br | H | $CH_2$ | Chinolin-2-yl | |
| 1.192 | H | $CH(CH_3)_2$ | $CH_2$ | Chinolin-2-yl | |
| 1.193 | H | H | $CH_2$ | 4-Nitro-chinolin-2-yl | |
| 1.194 | $CH_3$ | H | $CH_2$ | 4-Nitro-chinolin-2-yl | |
| 1.195 | $C_2H_5$ | H | $CH_2$ | 4-Nitro-chinolin-2-yl | |
| 1.196 | $(CH=CH)_2$ | | $CH_2$ | 4-Nitro-chinolin-2-yl | |

| No. | X | Y | A | Het | Smp. |
|---|---|---|---|---|---|
| 1.197 | Cl | H | $CH_2$ | 4-Nitro-chinolin-2-yl | |
| 1.198 | CH=CCl-CH=CH | | $CH_2$ | 4-Nitro-chinolin-2-yl | |
| 1.199 | Br | H | $CH_2$ | 4-Nitro-chinolin-2-yl | |
| 1.200 | H | $CH(CH_3)_2$ | $CH_2$ | 4-Nitro-chinolin-2-yl | |
| 1.201 | H | H | $CH_2$ | Chinoxalin-2-yl | |
| 1.202 | $CH_3$ | H | $CH_2$ | Chinoxalin-2-yl | |
| 1.203 | $C_2H_5$ | H | $CH_2$ | Chinoxalin-2-yl | |
| 1.204 | $(CH=CH)_2$ | | $CH_2$ | Chinoxal-2-yl | |
| 1.205 | Cl | H | $CH_2$ | Chinoxal-2-yl | |
| 1.206 | CH=CCl-CH=CH | | $CH_2$ | Chinoxal-2-yl | |
| 1.207 | Br | H | $CH_2$ | Chinoxal-2-yl | |
| 1.208 | H | $CH(CH_3)_2$ | $CH_2$ | Chinoxal-2-yl | |
| 1.209 | H | H | $CH(CH_3)CH_2$ | 4-Chlor-pyridin-2-yl | |
| 1.210 | $CH_3$ | H | $CH(CH_3)CH_2$ | 4-Chlor-pyridin-2-yl | |
| 1.211 | $C_2H_5$ | H | $CH(CH_3)CH_2$ | 4-Chlor-pyridin-2-yl | |
| 1.212 | (CH=CH) | H | $CH(CH_3)CH_2$ | 4-Chlor-pyridin-2-yl | |
| 1.213 | Cl | H | $CH(CH_3)CH_2$ | 4-Chlor-pyridin-2-yl | |
| 1.214 | CH=CCl-CH=CH | | $CH(CH_3)CH_2$ | 4-Chlor-pyridin-2-yl | |
| 1.215 | Br | H | $CH(CH_3)CH_2$ | 4-Chlor-pyridin-2-yl | |
| 1.216 | H | $CH(CH_3)_2$ | $CH(CH_3)CH_2$ | 4-Chlor-pyridin-2-yl | |
| 1.217 | H | H | $CH_2$ | 2,4-Dichlor-pyridin-6-yl | |
| 1.218 | $CH_3$ | H | $CH_2$ | 2,4-Dichlor-pyridin-6-yl | |
| 1.219 | $C_2H_5$ | H | $CH_2$ | 2,4-Dichlor-pyridin-6-yl | |
| 1.220 | $(CH=CH)_2$ | | $CH_2$ | 2,4-Dichlor-pyridin-6-yl | |
| 1.221 | Cl | H | $CH_2$ | 2,4-Dichlor-pyridin-6-yl | |
| 1.222 | CH=CCl-CH=CH | | $CH_2$ | 2,4-Dichlor-pyridin-6-yl | |
| 1.223 | Br | H | $CH_2$ | 2,4-Dichlor-pyridin-6-yl | |
| 1.224 | H | $CH(CH_3)_2$ | $CH_2$ | 2,4-Dichlor-pyridin-6-yl | |
| 1.225 | H | H | $CH_2$ | 4-Methoxy-pyrimidin-2-yl | |
| 1.226 | $CH_3$ | H | $CH_2$ | 4-Methoxy-pyrimidin-2-yl | |
| 1.227 | $C_2H_5$ | H | $CH_2$ | 4-Methoxy-pyrimidin-2-yl | |
| 1.228 | $(CH=CH)_2$ | | $CH_2$ | 4-Methoxy-pyrimidin-2-yl | |
| 1.229 | Cl | H | $CH_2$ | 4-Methoxy-pyrimidin-2-yl | |
| 1.230 | CH=CCl-CH=CH | | $CH_2$ | 4-Methoxy-pyrimidin-2-yl | |

| No. | X | Y | A | Het | Smp. |
|---|---|---|---|---|---|
| 1.231 | Br | H | $CH_2$ | 4-Methoxy-pyrimidin-2-yl | |
| 1.232 | H | $CH(CH_3)_2$ | $CH_2$ | 4-Methoxy-pyrimidin-2-yl | |
| 1.233 | H | H | $CH(CH_3)CH_2$ | Pyridin-4-yl | |
| 1.234 | $CH_3$ | H | $CH(CH_3)CH_2$ | Pyridin-4-yl | |
| 1.235 | $C_2H_5$ | H | $CH(CH_3)CH_2$ | Pyridin-4-yl | |
| 1.236 | $(CH=CH)_2$ | | $CH(CH_3)CH_2$ | Pyridin-4-yl | |
| 1.237 | Cl | H | $CH(CH_3)CH_2$ | Pyridin-4-yl | |
| 1.238 | CH=CCl-CH-CH | | $CH(CH_3)CH_2$ | Pyridin-4-yl | |
| 1.239 | Br | H | $CH(CH_3)CH_2$ | Pyridin-4-yl | |
| 1.240 | H | $CH(CH_3)_2$ | $CH(CH_3)CH_2$ | Pyridin-4-yl | |
| 1.241 | H | H | $CH_2$ | 4-Methoxy-pyrimidin-2-yl | |
| 1.242 | $CH_3$ | H | $CH_2$ | 4-Methoxy-pyrimidin-2-yl | |
| 1.243 | $C_2H_5$ | H | $CH_2$ | 4-Methoxy-pyrimidin-2-yl | |
| 1.244 | $(CH=CH)_2$ | | $CH_2$ | 4-Methoxy-pyrimidin-2-yl | |
| 1.245 | Cl | H | $CH_2$ | 4-Methoxy-pyrimidin-2-yl | |
| 1.246 | CH=CCl-CH=CH | | $CH_2$ | 4-Methoxy-pyrimidin-2-yl | |
| 1.247 | Br | H | $CH_2$ | 4-Methoxy-pyrimidin-2-yl | |
| 1.248 | H | $CH(CH_3)_2$ | $CH_2$ | 4-Methoxy-pyrimidin-2-yl | |
| 1.249 | H | H | $CH_2$ | Pyrimidin-2-yl | |
| 1.250 | $CH_3$ | H | $CH_2$ | Pyrimidin-2-yl | |
| 1.251 | $C_2H_5$ | H | $CH_2$ | Pyrimidin-2-yl | |
| 1.252 | $(CH=CH)_2$ | | $CH_2$ | Pyrimidin-2-yl | |
| 1.253 | Cl | H | $CH_2$ | Pyrimidin-2-yl | |
| 1.254 | CH=CCl-CH=CH | | $CH_2$ | Pyrimidin-2-yl | |
| 1.255 | Br | H | $CH_2$ | Pyrimidin-2-yl | |
| 1.256 | H | $CH(CH_3)_2$ | $CH_2$ | Pyrimidin-2-yl | |
| 1.257 | H | H | $CH(CH_3)$ | Pyridin-4-yl | |
| 1.258 | $CH_3$ | H | $CH_2$ | Pyridin-4-yl | |
| 1.259 | $C_2H_5$ | H | $CH_2$ | Pyridin-4-yl | |
| 1.260 | $(CH=CH)_2$ | | $CH_2$ | Pyridin-4-yl | |
| 1.261 | Cl | H | $CH_2$ | Pyridin-4-yl | |
| 1.262 | CH=CCl-CH=CH | | $CH_2$ | Pyridin-4-yl | |
| 1.263 | Br | H | $CH_2$ | Pyridin-4-yl | |

| No. | X | Y | A | Het | Smp. |
|---|---|---|---|---|---|
| 1.264 | H | $CH(CH_3)_2$ | $CH_2$ | Pyridin-4-yl | |
| 1.265 | H | H | $CH_2$ | 1,3,4-triazin-5-on-2-yl | |
| 1.266 | $CH_3$ | H | $CH_2$ | 1,3,4-triazin-5-on-2-yl | |
| 1.267 | $C_2H_5$ | H | $CH_2$ | 1,3,4-triazin-5-on-2-yl | |
| 1.268 | $(CH{=}CH)_2$ | | $CH_2$ | 1,3,4-triazin-5-on-2-yl | |
| 1.269 | Cl | H | $CH_2$ | 1,3,4-triazin-5-on-2-yl | |
| 1.270 | $CH{=}CCl{-}CH{=}CH_2$ | | $CH_2$ | 1,3,4-triazin-5-on-2-yl | |
| 1.271 | Br | H | $CH_2$ | 1,3,4-triazin-5-on-2-yl | |
| 1.272 | H | $CH(CH_3)_2$ | $CH_2$ | 1,3,4-triazin-5-on-2-yl | |
| 1.273 | $C_2H_5$ | H | $(CH_2)_3$ | 1-Phenyl-pyrazol-4-yl | 143-147° |
| 1.274 | $(CH{=}CH)_2$ | | $(CH_2)_3$ | 1H-Pyrazol-4-yl | fest |
| 1.275 | $(CH{=}CH)_2$ | | $(CH_2)_3$ | 1-Methyl-pyrazol-4-yl | 117-118° |
| 1.276 | $(CH{=}CH)_2$ | | $(CH_2)_3$ | 1-Phenyl-pyrazol-4-yl | 128-131° |
| 1.277 | $CH_3$ | H | $CH_2$ | Tetrahydropyran-2-yl | 106-109° |
| 1.278 | $CH_3$ | H | $(CH_2)CH_2$ | Pyrazol-1-yl | 102-104° |
| 1.279 | $C_2H_5$ | H | $CH_2CH_2$ | Pyrimidin-5-yl | 120-122° |
| 1.280 | $C_2H_5$ | H | $CH_2$ | 5-(4,5-Dihydro-3-methylthio-6-tert.-butyl-1,3-triazin-5-on-2-yl-hydrazin-oxim)-furfur-2-yl | Oel |

Formulierungsbeispiele:

Beispiel 2: Formulierungsbeispiele für Wirkstoffe der Formel I (% = Gewichtsprozent)

| a) Spritzpulver | a) | b) | c) |
|---|---|---|---|
| Wirkstoff gemäss Tabelle 1 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff gemäss Tabelle 1 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff gemäss Tabelle 1 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff gemäss Tabelle 1 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff gemäss Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff gemäss Tabelle 1 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff gemäss Tabelle 1 | 5 % |
| Isopropylamin | 1 % |
| Octylphenolpolyäthylenglykoläther (78 Mol AeO) | 3 % |
| Wasser | 91 % |

## Biologische Beispiele:

### Beispiel 3: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25°C und 50-70 % relativer Luftfeuchtigkeit gehalten und der Versuch nach 3 Wochen abgeschlossen und der Zustand der Pflanzen nach folgender Notenskala bewertet.

1 Pflanze hat nicht gekeimt oder ist eingegangen
2-3 sehr starke Schäden
4 starke Schäden
5 mittlere Schäden, die Pflanzen sind verkümmert
6 Schäden, die Pflanze kann regenerieren
7-8 leichte Schäden
9 normales Wachstum, wie das unbehandelter Pflanzen

In diesem Versuch zeigten die geprüften Verbindungen der Tabelle 1 starke Herbizidwirkung.

### Beispiel 4: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Auch in diesem Versuch zeigen die geprüften Verbindungen der Tabelle 1 starke bis sehr starke Herbizidwirkung.

Beispiel 5: Herbizidwirkung vor dem Auflaufen der Pflanzen

Kunststofföpfe werden mit expandiertem Vermiculit (Dichte: 0,135 g/cm³, Wasserabsorptionsvermögen: 0,565 l/l) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem in Beispiel 3 gegebenen Massstab bewertet.

Die Resultate sind in der Tabelle 2 zusammengefasst:

Tabelle 2

| Verb. No. | Aufwand Menge ppm | Nasturtium officinalis | Agrostis tenuis | Stellaria media | Digitaria sang. |
|---|---|---|---|---|---|
| 1.001 | 100 | 2 | 2 | 2 | 2 |
| | 10 | 2 | 2 | 2 | 2 |
| | 1 | 2 | 2 | 2 | 2 |
| | 0.1 | 3 | 3 | 3 | 3 |
| | 0.01 | 3 | 3 | 3 | 3 |
| 1.009 | 100 | 2 | 2 | 2 | 2 |
| | 10 | 2 | 2 | 2 | 2 |
| | 1 | 2 | 2 | 2 | 2 |
| | 0.1 | 3 | 3 | 3 | 3 |
| | 0.01 | 5 | 5 | 5 | 5 |
| 1.010 | 100 | 2 | 2 | 2 | 2 |
| | 10 | 2 | 2 | 2 | 2 |
| | 1 | 2 | 2 | 2 | 2 |
| | 0.1 | 3 | 3 | 3 | 4 |
| | 0.01 | 4 | 4 | 4 | 4 |
| 1.011 | 100 | 2 | 2 | 2 | 2 |
| | 10 | 2 | 2 | 2 | 2 |
| | 1 | 3 | 3 | 3 | 3 |
| | 0.1 | 4 | 4 | 3 | 3 |
| | 0.01 | 4 | 4 | 4 | 4 |
| 1.012 | 100 | 2 | 2 | 2 | 2 |
| | 10 | 2 | 2 | 2 | 2 |
| | 1 | 3 | 3 | 3 | 3 |
| | 0.1 | 4 | 4 | 4 | 4 |
| | 0.01 | 5 | 5 | 4 | 4 |
| A | 100 | 2 | 2 | 1 | 2 |
| | 10 | 2 | 2 | 2 | 2 |
| | 1 | 4 | 6 | 2 | 3 |
| | 0.1 | 9 | 9 | 7 | 7 |
| | 0.01 | 9 | 9 | 9 | 9 |

A ist der aus der EP-A 41623 bekannte 2-(5-Isopropyl-5-methyl-4-oxo-4,5-dihydroimidazol-2-yl)-5-methyl-nicotinsäure-äthylester.

Beispiel 6 Herbizidwirkung für Wasserreis (paddy rice)

Die Wasserunkräuter Echinochloa crus galli und Monocharia vag. werden in Plastikbechern (60 cm² Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt

3 Tage nach der Saat mit einer wässrigen Emulsion der Prüfsubstanzen durch eine Spritzung auf die Gefässe. Die Dosis entspricht einer Wirkstoffmenge von 4 kg AS pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt.

Die Bonitur erfolgt nach im Beispiel 3 gegebenen linearen Notenskala.

Die geprüften Verbindungen der Tabelle 1 zeigten in diesem Versuch gute Wirkung.

Beispiel 7: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabelle 1 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 8: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca.

5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Tabelle 1 eine merklicher Erhöhung der Anzahl und des Gewichts der Schoten im Haupttrieb.

Beispiel 9: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabelle 1 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 10: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgeleufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabelle 1 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Formel I bewirken eine Reduzierung des Neuzuwachses von um 10-30 % im Vergleich zur unbehandelten Kontrolle.

Beispiel 11: Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen des Wirkstoffes No. 1 in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

In diesem Versuch liessen die geprüften Verbindungen der Tabelle 1 bei Aufwandmengen von 0,6 und 1,2 kg/ha nach 7 Tagen bloss noch wenige vertrocknete Blätter auf den Pflanzen (> 80 % Blattfall und Austrocknung).

Patentansprüche

1. Heterocyclylalkylester von 2-Imidazolinon-nicotinsäuren der Formel I

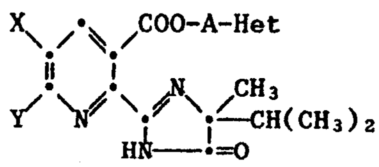

I

worin

A eine geradkettige oder verzweigte $C_1$-$C_4$-Alkylenbrücke

X und Y unabhängig voneinander je Wasserstoff $C_1$-$C_3$-Alkyl, $C_1$-$C_3$-Haloalkyl, $C_1$-$C_3$-Alkoxy , $C_1$-$C_3$-Haloalkoxy, Halogen, oder

X und Y bilden zusammen die Butadienbrücke CH=CH-CH=CH, die durch Halogen, Cyan oder $C_1$-$C_4$-Alkyl substituiert sein kann,

Het einen 5-6 gliedrigen , ein bis 3 Stickstoffatome und/oder ein Sauerstoff- oder Schwefelatom und gegebenenfalls eine oder 2 Carbonylgruppen enthaltenden Heterocyclus, der benzanneliert und/oder durch Halogen, $C_1$-$C_3$-Alkyl, $C_1$-$C_3$-Halogenalkyl, $C_1$-$C_3$-Alkoxy, $C_1$-$C_3$-Halogenalkoxy, Nitro, Amino, $C_1$-$C_3$-Alkylamino oder $C_1$-$C_3$-Dialkyamino substituiert sein kann oder den 2-(4,5-Dihydro-3-methylthio-6-tert.-butyl-1,3,4-triazin-5-on-2-ylhydrazinoxim)-furfuroxim-5-yl-Rest bedeuten, wobei die Bestimmung gilt, dass Het nicht der unsubstituierte Furylrest sein darf.

2. Heterocyclylalkylester von 2-Imidazolinon-nicotinsäuren der Formel I Anspruch 1, worin

A eine Methylen oder Aethylenbrücke, die durch Methyl substituiert sein kann,

X Wasserstoff oder $C_1$-$C_3$-Alkyl,

Y Wasserstoff und

Het die im Anspruch 1 gegebene Bedeutung hat.

3. Heterocyclylalkylester von 2-Imidazolinon-nicotinsäuren der Formel I Anspruch 1 worin

A die Methylen-, Aethyl-1-en oder Aethyl-2-en-brücke,

0215738

X Wasserstoff oder $C_1$-$C_3$-Alkyl

Y Wasserstoff und

Het einen ungesättigten 5-6-gliedrigen, ein bis drei Stickstoffatome und gegebenenfalls eine oder zwei Carbonylgruppen enthaltenden Heterocyclus, der benzannelliert und durch Methyl substituiert sein kann.

4. 2-(5-Isopropyl-5-methyl-4-oxo-4,5-dihydroimidazolin-2-yl)-5-methyl-nicotinsäure(4-pyridylmethyl)ester gemäss Anspruch 1.

5. 5-Aethyl-2-(5-isopropyl-5-methyl-4-oxo-4,5-dihydro-imidazolin-2-yl)-nicotinsäure-indolyläthyl-ester gemäss Anspruch 1.

6. 2-(5-Isopropyl-5-methyl-4-oxo-4,5-dihydroimidazolin-2-yl)-5-methyl-nicotinsäure-indolyläthyl-ester gemäss Anspruch 1.

7. 5-Aethyl-2-(5-isopropyl-5-methyl-4-oxo-4,5-dihydro-imidazolin-2-yl)-nicotinsäure-pyridin-2-yläthylester gemäss Anspruch 1.

8. 5-Aethyl-2-(5-isopropyl-5-methyl-4-oxo-4,5-dihydro-imidazolin-2-yl)-nicotinsäure-isoindol-2-yläthyl-ester gemäss Anspruch 1.

9. 5-Aethyl-2-(5-isopropyl-5-methyl-4-oxo-4,5-dihydro-imidazolin-2-yl)-nicotinsäure-pyridin-2-ylmethyl-ester gemäss Anspruch 1.

10. 5-Aethyl-2-(5-isopropyl-5-methyl-4-oxo-4,5-dihydro-imidazolin-2-yl)-nicotinsäure-pyridin-3-ylmethyl-ester gemäss Anspruch 1.

11. 5-Aethyl-2-(5-isopropyl-5-methyl-4-oxo-4,5-dihydro-imidazolin-2-yl)-nicotinsäure-pyridin-4-ylmethyl-ester gemäss Anspruch 1.

12. 2-Isopropyl-5-methyl-4-oxo-4,5-dihydro-imidazolin-2-yl)-5-methyl-nicotinsäure-pyridin-2-ylmethyl-ester gemäss Anspruch 1.

13. 2-(5-Isopropyl-5-methyl-4-oxo-4,5-dihydro-imidazolin-2-yl)-5-methyl-nicotinsäurepyridin-3-ylmethyl)-ester gemäss Anspruch 1.

14. 2-(5-Isopropyl-5-methyl-4-oxo-4,5-dihydro-imidazolin-2-yl)-nicotinsäure-pyridin-2-yläthyl-ester gemäss Anspruch 1.

15. 2-(5-Isopropyl-5-methyl-4-oxo-4,5-dihydro-imidazolin-2-yl)-5-methyl-nicotinsäure-pyridin-2-yläthylester gemäss Anspruch 1.

16. 5-Aethyl-2-(5-isopropyl-5-methyl-4-oxo-4,5-dihydro-imidazolidin-2-yl)-nicotinsäure-pyridin-2-yl-äthyl[2-(4,5-dihydro-3-methylthio-6-tert.-butyl-1,3,4-triazin-5-on-2-yl-hydrazinoxim)-furfur-5-yl]-ester gemäss Anspruch 1.

17. Verfahren zur Herstellung der Heterocyclylalkylester von 2-Imidazolinon-nicotin-säuren der Formel I, Anspruch 1, dadurch gekennzeichnet, dass man eine Verbindung der Formel IIa resp. IIb

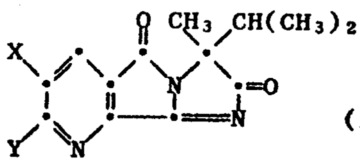 (IIa) 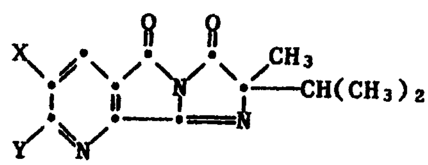 (IIb)

worin X und Y die im Anspruch 1 gegebene Bedeutung haben, in einem inerten organischen Lösungs- oder Verdünnungsmittel in Gegenwart eines basischen Mittels, mit einer Verbindung der Formel III umsetzt,

HO-A-Het (III)

worin A und Het die im Anspruch 1 gegebene Bedeutung haben.

18. Ein herbizides und den Pflanzenwachstum regulierendes Mittel, dadurch gekennzeichnet, dass es neben Träger- und/oder anderen Zuschlagstoffen als Wirkstoff einen Heterocyclylalkylester einer 2-Imidazolinon-nicotinsäure gemäss Annspruch 1 enthält.

19. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge einer Verbindung der Formel I, Anspruch 1 oder eines eine solche Verbindung enthaltendes Mittel behandelt.

20. Verfahren zur Hemmung und Unterdrückung des Pflanzenwachstums über das 2-Blattstadium hinaus, dadurch gekennzeichnet, dass man die Pflanzen während ihres Wachstums mit einer wirksamen Menge einer Verbindung der Formel I Anspruch 1 oder eines einen solchen Wirkstoff enthaltenden Mittels behandelt.

21. Verfahren zur Dessication und Defoliation von Baumwoll- und Kartoffelkulturen zur Erleichterung deren Ernte dadurch gekennzeichnet, dass man die Kultur kurz vor der Ernte mit einer wirksamen Menge einer Verbindung der Formel I, Anspruch 1 oder eines einen solchen Wirkstoff enthaltenden Mittels behandelt.

FO 7.5 NU/eg*

Europäisches Patentamt

**EUROPÄISCHER RECHERCHENBERICHT**

0215738

Nummer der Anmeldung

EP 86810400.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A2 - 0 041 624 (AMERICAN CYANAMID)<br>* Seite 86, Zeile 4; Ansprüche * | 1,17-20 | C 07 D 401/14<br>C 07 D 403/14<br>C 07 D 405/14<br>C 07 D 409/14<br>C 07 D 413/14<br>C 07 D 417/14<br>A 01 N 43/50 |
| D,X | EP - A2 - 0 041 623 (AMERICAN CYANAMID)<br>* Ansprüche * | 1,17-20 | |
| X | US - A - 4 404 012 (ORWICK)<br>* Spalten 25-26; Ansprüche * | 1,17-20 | |
| | | | **RECHERCHIERTE SACHGEBIETE (Int. Cl.4)** |
| | | | C 07 D 401/00<br>C 07 D 403/00<br>C 07 D 405/00<br>C 07 D 409/00<br>C 07 D 413/00<br>C 07 D 417/00 |

Der vorliegende Recherchenbericht wurde für alle Patentansprüche erstellt.

| Recherchenort | Abschlußdatum der Recherche | Prüfer |
|---|---|---|
| WIEN | 03-12-1986 | HAMMER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82